# EUROPÄISCHE PATENTSCHRIFT

(11) **EP 4 446 723 B1**
(45) Veröffentlichungstag und Bekanntmachung des Hinweises auf die Patenterteilung: **05.08.2026**
(21) Anmeldenummer: 23167626.3
(22) Anmeldetag: 12.04.2023
(51) Int. Cl.: G01N 33/483, G01N 1/30

(54) **SCHONENDES VERFAHREN ZUR HERSTELLUNG TRANSPARENTER BIOLOGISCHER PRÄPARATE FÜR EINE LICHTMIKROSKOPISCHE UNTERSUCHUNG**
GENTLE METHOD FOR PRODUCING TRANSPARENT BIOLOGICAL PREPARATIONS FOR A LIGHT MICROSCOPIC EXAMINATION
PROCÉDÉ DOUX POUR LA PRÉPARATION DE PRÉPARATIONS BIOLOGIQUES TRANSPARENTES POUR L'EXAMEN PAR MICROSCOPIE OPTIQUE

(43) Veröffentlichungstag der Anmeldung: 16.10.2024
(73) Patentinhaber: Georg-August-Universität Göttingen Stiftung Öffentlichen Rechts, Universitätsmedizin, 37075 Göttingen (DE)
(72) Erfinder: Wouters-Bunt, Fred, Göttingen (DE); Bunt, Gertrude, Göttingen (DE)
(74) Vertreter: Patentanwälte Olbricht Buchhold Keulertz

(56) Entgegenhaltungen:
- WO-A1-2022/129222
- US-A1- 2013 015 067
- US-A1- 2019 302 053

## Beschreibung

Die Erfindung betrifft ein Verfahren gemäß dem Oberbegriff von Anspruch 1.

Transparente biologische Gewebeproben werden benötigt, um die Gewebeproben zum Beispiel mittels Lichtblattmikroskopie dreidimensional abbilden zu können. Um die Transparenz von biologischen Präparaten zu erreichen, sind insbesondere Häm-Gruppen des Blutfarbstoffs Hämoglobin und Lipide aus den biologischen Präparaten zu entfernen. Mittels z.B. einer stark basischen Vorbehandlung erfolgt die Freisetzung von Gewebekomponenten, die die Durchsichtigkeit einschränken, z.B. Häm-Verbindungen und Lipide. Die so freigesetzten Gewebekomponenten werden anschließend aktiv mittels Elektrophorese aus dem Gewebe entfernt. In dem weiteren Verlauf des "Elektroclearings"-Prozesses wird das Gewebe typischerweise in einer Alkoholreihe entwässert, bevor es zur Brechungsindex-Anpassung in ein passendes, beispielsweise organisches, Lösungsmittel gegeben wird.

Die US 2005/0130317 A1 beschreibt eine Vorrichtung zur parallelen Analyse von biologischen Molekülen mit einem Reaktionsraum, der sich zwischen zwei Elektroden erstreckt. An die Elektroden wird eine Spannung angelegt, die zu einem Wandern der zwischen die Elektroden eingebrachten biologischen Moleküle führt.

Bei dem Gewebeclearingverfahren gemäß DE 10 2016 123 458 B3 wird das fixierte Gewebe nach einer stark basischen Vorbehandlung bei pH>14 mit einer Alkalimetallhydroxid-Lösung in einer ebenfalls basischen Lösung bei typischerweise 8<pH<10 elektrophoretisiert. Einige Gewebetypen, insbesondere Gehirn und Lunge, quellen während der basischen Elektrophorese auf.

Während der Entwässerung ist, wie aus der histologischen Praxis bekannt, generell ein Schrumpfen des fixierten Gewebes zu beobachten. Während das Schrumpfen von Gewebe für die weitere Analyse also gut toleriert werden kann, stellt ein Aufquellen eine größere strukturelle Belastung für das Gewebematerial dar. Durch den Zyklus von Aufquellen und Schrumpfen entsteht das Problem, dass empfindliche Gewebeprobe eine mechanische Beschädigung erleiden. Wenn das Gewebe keine ausreichende elastische Verformung zulässt oder z.B. in einer nichtelastischen Kapsel eingebettet ist, können Risse entstehen. Oft treten die mechanischen Strukturfehler erst oder verstärkt auf, wenn das Gewebe nach einem vorangegangenen Aufquellen (nach einer Vergrößerung) anschließend wieder schrumpft. Die hierbei auftretende Erhärtung des Materials und die Kräfte, die sich bei der Verformung entwickeln, greifen das Gewebe offensichtlich besonders stark an. Proben mit einer flachen Geometrie, also mit einer geringen Ausbreitung in eine der drei Raumdimensionen, scheinen hierbei besonders gefährdet. Nach der Entwässerung haben die Gewebeproben ihre kleinste Ausdehnung. Während aller Behandlungsschritte bis einschließlich des Entwässerns soll das Gewebe möglichst kontrolliert schrumpfen und dabei insbesondere ein Aufquellen des Gewebes aus den oben genannten Gründen verhindert werden. Für die empfindlichen Gewebeproben ist es deshalb wünschenswert, dass auch die Elektrophorese zumindest ohne Aufquellen abläuft.

Der Erfindung liegt die Aufgabe zugrunde, ein Verfahren zur Herstellung transparenter Gewebeproben eines biologischen Gewebes zur lichtmikroskopischen Untersuchung bereitzustellen, das die vorstehenden Nachteile überwindet und das insbesondere eine zuverlässige Klärung der Gewebeproben ohne eine Beschädigung oder Beeinträchtigung der Gewebeproben ermöglicht und möglichst den Aufwand für den Klärungsvorgang und die Probenaufbereitung reduziert.

Die Aufgabe wird erfindungsgemäß gelöst durch ein Verfahren gemäß Patentanspruch 1.

Weitere Ausführungsformen sind Gegenstand der Unteransprüche oder nachfolgend beschrieben.

Das erfindungsgemäße Verfahren zur Herstellung transparenter Gewebeproben eines biologischen Gewebes zur lichtmikroskopischen Untersuchung, umfasst die Schritte:
a) Inkubation des Gewebes in einer stark basischen Lösung, bevorzugt in einer Alkalimetallhydroxidlösung, und
b) Klären der entwässerten Gewebeprobe durch Elektrophorese, indem das Gewebe in eine wässrige Elektrophoreselösung eingetaucht und in der Elektrophoreselösung einem elektrischen Feld ausgesetzt wird.

Die Elektrophoreselösung enthält eine Puffersubstanz in einer Konzentration von 1 bis 100 mol/m³ und mindestens ein nichtionisches Detergens in einer Konzentration von 0,1 bis 10 Gew.-%. Denkbar ist auch eine Konzentration von 1 bis 5 Gew.-%. Die Elektrophoreselösung weist einen pH-Wert 4.5-7.9 auf. Die Puffersubstanz ist eine organische Verbindung mit mindestens einer Carbonsäuregruppe oder mindestens einer Sulfonsäuregruppe und weist mindestens eine Amingruppe auf. Der pH-Wert der Elektrophoreselösung ergibt sich durch die Einbringung der Substanz in die wässrige Lösung, d.h. dass der pH-Wert ohne Zugabe von starken Säuren oder Basen gestellt wird.

Die Inkubation des Gewebes in Schritt a) erfolgt bevorzugt in einer Alkalimetallhydroxidlösung, die neben Alkalimetallhydroxid Ethanol und mindestens ein nichtionisches Detergens enthält. Bei dieser Inkubation erweist es sich als günstig, wenn folgende Parameter zumindest teilweise eingehalten werden:
Das Inkubieren erfolgt für einen Zeitraum von 30 bis 120 min, vorzugsweise von 45 bis 90 min. Das Inkubieren erfolgt bei einer Temperatur von 20 bis 50 °C, vorzugsweise von 35 bis 40 °C, das heißt von etwa 37,5 °C. Die wässrige alkalische Lösung weist eine Alkalikonzentration von 50 bis 2.000 mol/m³, vorzugsweise von 100 bis 1.000 mol/m³ auf. Die wässrige alkalische Lösung weist NaOH zur Bereitstellung ihrer Alkalikonzentration auf. Die wässrige alkalische Lösung weist einen C1-5-Alkohol in einer Konzentration von 10 bis 70 Volumen-% oder vorzugsweise von 40 bis 60 Volumen-% (v/v) auf. Die wässrige alkalische Lösung weist Ethanol auf und die wässrige alkalische Lösung weist ein Detergens in einer Konzentration von 0,1 bis 10 Gew.-% und vorzugsweise von 0,5 bis 2,5 Gew.-% auf.

In einer bevorzugten Ausführungsform des erfindungsgemäßen Verfahrens hat die Elektrophoreselösung einen pH-Wert von pH 4.5 bis 7.9, bevorzugt in einem Bereich zwischen 5.0 und 7.9, weiter bevorzugt einen pH-Wert von 5.5 bis 7.9. Bevorzugt stellt sich der pH-Wert der Elektrophoreselösung in Wasser spontan ein, d.h. es wird keine zusätzliche starke Säure oder Base zugesetzt, um den pH abweichend vom spontanen pH einzustellen. Starke Säuren sind in diesem Sinne als die Untergruppe der Säuren zu verstehen, die eine Säurekonstante (pKs) <=4,5 aufweisen. Starke Basen sind in diesem Sinne als die Untergruppe der Basen zu verstehen, die eine Basenkonstante (pKb) <=4,5, dieses entspricht einer Säurekonstanten (pKa)>= 9.5, aufweisen. Starke Säuren und Basen liegen in wässrige Lösung zu einem erheblichen Teil ionisiert vor, und erhöhen die Leitfähigkeit der Elektrophoreselösung unnötig. Die Elektrophoreselösung ist dann frei von pH-Stellmitteln, die die Leitfähigkeit unnötig erhöhen. Grundsätzlich ist es vorteilhaft, wenn die Leitfähigkeit so niedrig wie möglich ist.

Die Konzentration der Puffersubstanz in der Elektrophoreselösung beträgt bevorzugt 5 bis 50 mol/m³ und mehr bevorzugt 15 bis 25 mol/m³.

Die Konzentration des nichtionischen Detergens in der Elektrophoreselösung beträgt vorzugsweise 0,5 bis 2,5 Gew.-%.

### Puffersubstanzen

Die Puffersubstanz hat bevorzugt eine molare elektrische Leitfähigkeit von kleiner als 2 Siemens·cm²/mol. Hierzu wird die spezifische Leitfähigkeit mittels eines Konduktometers in einer Verdünnungsreihe bei geringer Konzentration (z.B. 5, 10, 20 und 40 mM) bestimmt. Die Messwerte in µS/cm werden in Abhängigkeit der Konzentration in mM aufgetragen. In diesem Verdünnungsbereich sollte die spezifische Leitfähigkeit eine lineare Abhängigkeit von der Konzentration besitzen. Diese Abhängigkeit wird anschließend mittels linearer Regression ermittelt. Die Neigung der gefundene Gerade stellt die molare elektrische Leitfähigkeit in Siemens·cm²/mol dar.

Bevorzugt werden leicht saure bis neutrale Puffersubstanzen ausgewählt, die als Reinstofflösung eine niedrige spontane Leitfähigkeit aufweisen. Diese Puffersubstanzen haben bevorzugt eine maximale Obergrenze der molaren Leitfähigkeit von 1.5 S cm²/mol, besonders bevorzugt 1 S cm²/mol.

Die Puffersubstanz ist bevorzugt eine organische Verbindung, die genau eine ionisierbare Säure-Gruppe enthält, die pro Molekül ein Proton erzeugen kann, oder die genau eine ionisierbare Base-Gruppe enthält, die pro Molekül ein Hydroxyl-Ion erzeugen kann, und die pro Molekül maximal eine positive und maximal eine negative Ladung und keine weiteren geladenen Gruppen enthält bei dem pH, der durch ihre Lösung in Wasser spontan entsteht. Besonders bevorzugt ist die Puffersubstanz eine organische Verbindung mit genau einer Carbonsäuregruppe oder genau einer Sulfonsäuregruppe und weist mindestens eine Amingruppe auf.

Die Puffersubstanz ist eine organische Verbindung mit mindestens einer Carbonsäuregruppe oder mindestens einer Sulfonsäuregruppe und weist mindestens eine Amingruppe auf. Bevorzugt ist die Pfuffersubstanz ausgewählt aus der Gruppe bestehend aus Aminocarbonsäuren, Aminosulfonsäuren, Aminocarbonsäuresalz oder einer Mischung hiervon. Weiter bevorzugt ist die Puffersubstanz eine Amino-Alkansulfonsäure, eine Hydroxyalkylaminosulfonsäure, eine Piperidin-Alkansäure, eine Aminosäure, eine N-Hydroxy-Alkyl-Aminocarbonsäure oder ein Aminocarbonsäuresalz oder Amino-Sulfonsäuresalze oder eine Mischung hiervon und weist eine molare elektrische Leitfähigkeit von kleiner als 2 Siemens·cm²/mol, gemessen nach dem oben genannten Verdünnungs-Verfahren, auf.

### Amino-Sulfonsäuren

Die Puffersubstanz kann aus der Gruppe der Amino-Alkansulfonsäuren gemäß der allgemeinen Formel I ausgewählt sein: 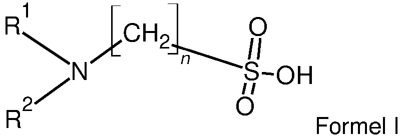 mit
n gleich 1 bis 4.
R¹ gleich Wasserstoffatom oder Methylgruppe und
R² gleich ein Cyclohexylrest, Alkylrest mit 1 bis 4 Kohlenstoffatomen oder Hydroxylalkylrest mit 1 bis 4 Kohlenstoffatomen und mit mindestens einer Hydroxylgruppe.

Geeignete Puffersubstanzen in Form von Amino-Alkansulfonsäure sind beispielsweise 2-Cyclohexylamino- Alkansulfonsäuren mit der Summenformel C₆H₁₁-NH-[CH₂]₁-SO₂OH mit n = 1 bis 4, gemäß Formel II 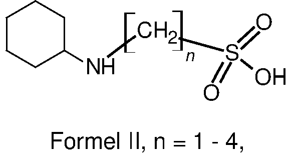 wie 2-(Cyclohexylamino)ethansulfonsäure (CHES) (CAS-Nummer) 103-47-9, 3-(Cyclohexylamino)-1-propansulfonsäure (CAPS) (CAS-Nummer) 1135-40-6 und 4-(Cyclohexylamino)-1-butansulfonsäure (CABS) (CAS-Nummer) 161308-34-5.

Weitere geeignete Amino-Alkansäuren sind Hydroxyalkylaminosulfonsäuren, beispielsweise Tris(hydroxymethyl)methylamino-Alkansulfonsäuren der Summenformel C[COH]₃-NH-[CH₂]ₙ-SO₂OH mit n = (1-4), wie z.B. N-Tris(hydroxymethyl)methyl-3-aminopropansulfonsäure (TAPS) (CAS-Nummer 29915-38-6) gemäß Formel III

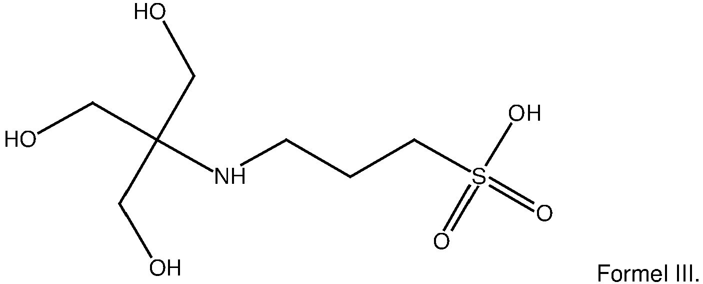

### Amino-Carbonsäuren

Weitere geeignete Puffersubstanzen für das erfindungsgemäße Verfahren sind Aminocarbonsäuren.

Geeignete Puffersubstanzen in Form von Aminocarbonsäuren sind beispielsweise nicht proteinogene Aminosäuren wie Piperidincarbonsäuren (Piperidinalkansäuren) mit der Summenformel C₆H₉NH-[CH₂]n-COOH mit n = 0-3 gemäß der Formel IV 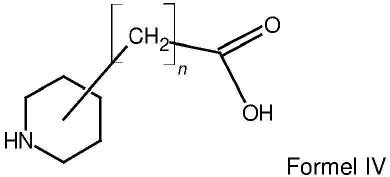
mit n gleich 0 bis 3 wie z.B.
Piperidin-2-Carbonsäure, auch Pipecolinsäure genannt (CAS-Nummer 535-75-1), mit n = 0, Piperidin-3-Carbonsäure, auch Nipecotinsäure genannt, (CAS-Nummer 498-95-3), mit n = 0 und
Piperidin-Essigsäure mit n = 1, wie Piperidin-2-Essigsäure und Piperidin-4-essigsäure, Piperidin-Propionsäure mit n = 2 und Piperidin-Butansäure mit n = 3.

Weitere als Puffersubstanz geeignete Aminocarbonsäuren sind Aminosäuren der Formeln NH₂-CHR-COOH und di-peptide NH₂-CHR-CONH-CHR-COOH, beispielweise Aminoessigsäure, auch Glycin genannt, (CAS-Nummer 56-40-6) gemäß der folgenden Formel 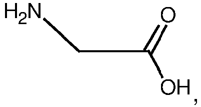 Glycylglycin, (CAS-Nummer 556-50-3) gemäß Formel V 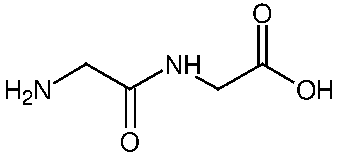 Formel V und Pyrrolidin-2-Carbonsäure, auch Prolin genannt (CAS-Nummer 609-36-9) gemäß Formel VI

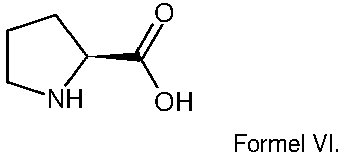

Auch N-Hydroxy-Alkyl-Aminocarbonsäure sind als Puffersubstanzen geeignet. Eine Gruppe dieser Aminosäuren bilden Tris(hydroxymethyl)methylaminocarbonsäuren mit der Summenformel C[COH]₃-NH-[CH₂]ₙ-COOH, mit n = (1-3), sind geeignete Puffersubstanzen. Eine geeignete Tris(hydroxymethyl)methylaminocarbonsäure ist beispielsweise N-[Tris(hydroxymethyl)methyl]aminoessigsäure, auch Tricin oder N-[Tris-(hydroxymethyl)-methyl]-glycin genannt (CAS-Nummer 5704-04-1) gemäß Formel VII

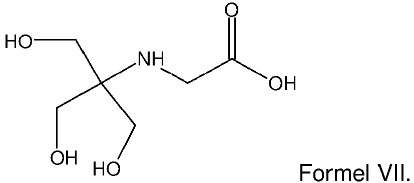

Ein weitere geeignete N-Hydroxyl-Alkyl-Aminosäure ist Bis(hydroxyethyl)aminoessigsäure, auch N,N-Bis-(2-hydroxyethyl)-glycin genannt (CAS-Nummer 150-25-4) gemäß Formel VIII

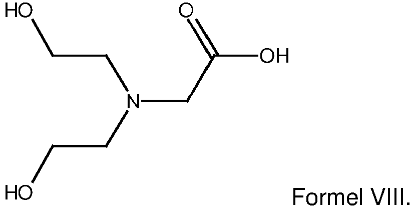

### Salze

Weitere geeignete Puffersubstanzen für das erfindungsgemäße Verfahren sind Aminocarbonsäuresalz und Amino-Sulfonsäuresalze.

Geeignete Aminocarbonsäuresalze sind die Gruppe der Trialkylammonio Alkansäuresalze, wie Trimethylammonio Alkansäuresalze mit der Summenformel N⁺ (CH₃)₃-[CH₂]ₙ-COO⁻ mit n = (1-3). Geeignete Trimethylammonio Alkansäuresalze sind z.B. Trimethylammonio-Acetat, auch Trimethylglycine, Betain genannt, (CAS-Nummer 107-43-7) gemäß Formel IX 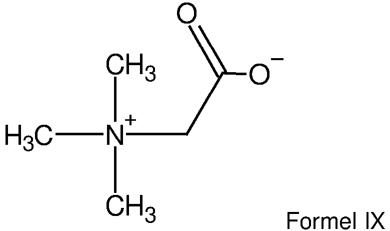 und Aminosäure-Salze mit längere Alkansäureketten wie Trimethylammonio-Propionat (n = 2) oder-Butyrat (4C). Auf Grund der ständig geladenen Stickstoffatome sind diese Verbindungen Salze.

Neben mit Methylgruppe N-substituierten Ammonio-Alkansäuresalzen sind auch Ethyl- substituierte oder mit höheren Alkylgruppen N-substituierte Salze geeignet.

Neben Trialkylammonio-Alkansäuresalzen können auch die strukturell ähnlichen Trialkylammonio-Alkylsulfonate, also Amino-Sulfonsäuresalze verwendet werden. Eine geeignetes Alkylammonio-Alkylsulfonat ist z.B. 3-[Dimethyl-(2-hydroxyethyl)ammonio]-1-propansulfonat (NDSB 211), CAS-Nummer 38880-58-9 gemäß Formel X:

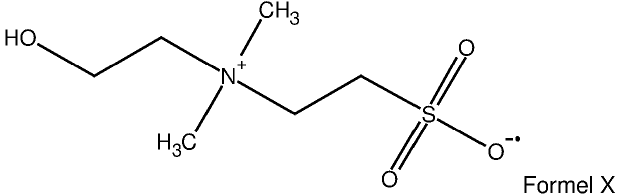

Ein weiteres geeignetes Alkylammonio-Alkylsulfonsäure ist N-(1,1-Dimethyl-2-hydroxyethyl)-3-amino-2-hydroxypropansulfonsäure (AMPSO), CAS-Nummer 68399-79-1 gemäß Formel XI

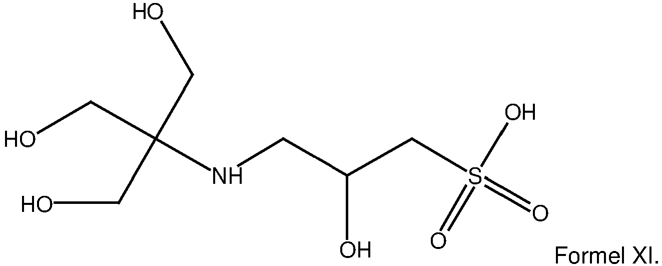

Überraschenderweise können die Puffersubstanzen für die elektrophoretische Klärung nicht nur als Reinsubstanz, sondern auch in Mischung mit anderen Puffersubstanzen oder Pufferbaseneingesetzt werden. Beispielweise können zwei der erfindungsgemäß geeigneten Puffersubstanzen gemischt werden. Eigene Versuche haben gezeigt, dass z.B. bei einer Mischung von N-Tris(hydroxymethyl)methyl-3-aminopropansulfonsäure (TAPS) und 3-(Cyclohexylamino)-1-pro-pansulfonsäure (CAPS), im Verhältnis 1:1 gemischt, die Leitfähigkeit des Gemisches identisch mit der der reinen CAPS-Lösung ist, obwohl TAPS an sich eine höhere Leitfähigkeit besitzt.

Ebenso können die Puffersubstanzen mit einer Pufferbase gemischt werden, wobei die Mischungen bevorzugt mehr als 50 Gew.-%, besonders bevorzugt mehr als 75 Gew.-% leicht saure bis neutrale Puffersubstanz enthalten, d.h. die Puffersubstanz die Hauptkomponente im Vergleich zur Pufferbase ist.

Die Puffersubstanz ist bevorzugt ausgewählt aus der Gruppe bestehend aus Amino-Alkansulfonsäuren gemäß der allgemeinen Formel I, Tris(hydroxymethyl)methylamino-Alkansulfonsäuren der Summenformel C[COH]₃-NH-[CH₂]ₙ-SO₂OH mit n = (1-4), Piperidincarbonsäuren mit der Summenformel C₆H₉NH-[CH₂]n-COOH mit n = 0-3, Aminosäuren der Formeln NH₂-CHR-COOH und di-peptide NH₂-CHR-CONH-CHR-COOH, N-Hydroxy-Alkyl-Aminocarbonsäure, Tris(hydroxymethyl)methylaminocarbonsäuren mit der Summenformel C[COH]₃-NH-[CH₂]ₙ-COOH, mit n = (1-3), N-Hydroxyl-Alkyl-Aminosäuren, Trialkylammonio Alkansäuresalze, wie Trimethylammonio Alkansäuresalze mit der Summenformel N⁺ (CH₃)₃-[CH₂]ₙ-COO⁻ mit n = (1-3), Trialkylammonio-Alkylsulfonate

Besonders bevorzugt ist die Puffersubstanz ausgewählt aus der Gruppe bestehend aus 2-(Cyclohexylamino)ethansulfonsäure, 3-(Cyclohexylamino)-1-propansulfonsäure, 4-(Cyclohexylamino)-1-butansulfonsäure, N-Tris(hydroxymethyl)methyl-3-aminopropansulfonsäure, Piperidin-2-Carbonsäure, Piperidin-3-Carbonsäure, Piperidin-Essigsäure, Piperidin-Propionsäure, Aminoessigsäure, Glycylglycin, Pyrrolidin-2-Carbonsäure, N-[Tris(hydroxymethyl)methyl]aminoessigsäure, Bis(hydroxyethyl)aminoessigsäure, Trimethylammonio-Acetat, Trimethylammonio-Proprionat, Trimethylammonio-Butyrat, 3-[Dimethyl-(2-hydroxyethyl)ammonio]-1-propansulfonat, N-(1,1-Dimethyl-2-hydroxyethyl)-3-amino-2-hydroxypropansulfonsäure und Mischungen hiervon.

### Detergenzien

Im erfindungsgemäßen Verfahren werden nichtionische Detergenzien eingesetzt. Nichtionische Detergenzien sind grenzflächenaktive Stoffe (Tenside) mit einer ungeladenen, im neutralen pH-Bereich keine lonenladung tragenden, polaren, hydrophilen, wasserlöslich machenden Kopfgruppe. Es versteht sich, dass die Angabe "nichtionisches Detergens" nicht so zu verstehen ist, dass hiermit nur ein Detergens mit einer lonizität von null gemeint ist. Vielmehr bezieht sich die Angabe auf alle Detergenzien, die keine ausgeprägte lonizität, das heißt zumindest im Wesentlichen keine lonizität aufweisen, insbesondere Detergenzien, die in einem pH-Bereich von pH > 4.5 keine Nettoladung pro Molekül besitzen. Vorzugsweise wird das nichtionische Detergens zudem danach ausgewählt, dass es eine möglichst geringe Affinität zu Proteinen aufweist. Geeignete nichtionische Detergenzien sind beispielweise Alkylphenyl-Ethoxylate, wie Octylphenyl-Ethoxylate, Fettsäureethoxylate, Fettalkoholpolyglycolether, Alkylpolyglucoside, sekundäre Alkohol-Ethoxylate, Betaine und Alkylsulfobetaine, auch Sultaine genannt, sowie Mischungen der Detergentien.

Praktische Erprobungen des erfindungsgemäßen Verfahrens haben gezeigt, dass zumindest die folgenden Detergentien als nichtionische Detergentien im erfindungsgemäßen Verfahren geeignet sind:
- Polyoxyethylen(20)-sorbitan-monolaurat (CAS-Nummer 9005-64-5), Handelsname Tween 20,
- Polyoxyethylensorbitan-monooleat (CAS-Nummer 9005-65-6), Handelsname Tween 80,
- Polyethylenglycol 4-tert-octylphenylether (Formel t-Oct-C₆H₄-(OCH₂CH₂)ₓOH, x= ~5, CAS-Nummer 9002-93-1), Handelsname Triton X45,
- Octylphenoxypolyethoxyethanol (Formel *t*-Oct-C₆H₄-(OCH₂CH₂)ₓOH, x= 9-10, CAS-Nummer 9036-19-5), Handelsname Triton X100,
- Polyethylenglycol-tert-octylphenylether (CAS Nummer 9036-19-5), Handelsname Triton X102,
- Polyethylenglycol monoalkylether (CAS Nummer 9043-30-5), Handelsname Genapol,
- n-octyl-beta-D-Glucopyranosid (CAS Nummer 29836-26-8),
- Decaethylenglykol-monododecylether (CAS Nummer 9002-92-0), Handelsname Brij,
- Octylphenoxypolyethylenoxyethanol (Formel (C₂H₄O)ₙC₁₄H₂₂O, CAS Nummer 9002-93-1), Handelsname IGEPAL CA-630,
- Sekundäre Alkohol-Ethoxylate (CAS Nummer 84133-50-6), Handelsname Tergitol, und
- 3-[(3-Cholamidopropyl)dimethylammonio]-1-propansulfonathydrat CAS Nummer 75621-03-3), Handelsname CHAPS,
- Lauryl Sulfobetaine (CAS Nummer 14933-08-5).

Besonders gute Eigenschaften zur Verwendung bei der vorliegenden Erfindung zeigen die nichtionischen Detergentien Polyoxyethylensorbitan-monooleat (Tween 80), Octylphenoxypolyethoxyethanol (Triton X100), Polyethylenglycol 4-tert-octylphenylether (Triton X45), Polyethylenglycol-tert-octylphenylether (Triton X102), Polyethylenglycol monoalkylether (Genapol), n-octylbeta-D-Glucopyranosid, Decaethylenglykol-monododecylether (Brij), Octylphenoxypolyethylenoxyethanol (IGEPAL CA-630) und sekundäre Alkohol-Ethoxylate (Tergitol). Die günstigsten Eigenschaften zeigen Octylphenoxypolyethoxyethanol (Triton X100), Decaethylenglykol-monododecylether (Brij), sekundäre Alkohol-Ethoxylate (Tergitol), Polyethylenglycol monoalkylether (Genapol) und n-octyl-beta-D-Glucopyranosid.

Das nichtionische Detergens, das bei der Elektrophorese verwendet wird, kann auch aus mehr als einem der voranstehend genannten Detergentien zusammengesetzt sein. Die Auswahl und/oder die Mischung der Detergentien kann gezielt auf verschiedene beim elektrophoretischen Klären zu entfernenden Inhaltsstoffe des jeweiligen biologischen Gewebes abgestimmt sein.

Durch die Verwendung eines nichtionisches Detergens bei dem erfindungsgemäßen Verfahren tritt kein durch das angelegte elektrische Feld hervorgerufener elektrischer Strom in Form von Detergens-Ionen auf, der als solcher in Bezug auf die angestrebte Klärung des biologischen Gewebes nur parasitär wäre. Die Folge solcher parasitärer lonenströme wäre unter anderem eine Erwärmung des biologischen Gewebes, ohne dass diese Erwärmung mit einer Klärung des biologischen Gewebes verbunden wäre. Parasitäre Ströme würden auch verhindern, dass elektrische Felder größerer Feldstärke über das biologische Gewebe hinweg ausgebildet werden können, weil die dadurch hervorgerufenen großen Ströme das biologische Gewebe zu sehr aufheizen würden. Solche elektrischen Felder größerer Feldstärke sind aber für das elektrische Herausleiten auch größerer und entsprechend unbeweglicher Mizellen aus dem biologischen Gewebe vorteilhaft.

Das erfindungsgemäße Verfahren umfasst bevorzugt die Schritte
0) Fixieren einer Gewebeprobe in Formaldehyd, Glutaraldehyd oder einem anderen vernetzenden Fixativ,
a) Vorbehandeln in einer Lösung mit Alkalimetallhydroxid,
b) Elektrophorese in einer Lösung mit niedrig konzentrierter Puffersubstanz, die ohne Beimischung einer weiteren starken Lauge oder Base einen pH-Wert zwischen 4.5 und 7.9, bevorzugt einen pH-Wert zwischen 5.0 und 7.9, weiter bevorzugt zwischen 5.5 und 7.9, aufweist, in Gegenwart eines nicht-ionischen Detergens,
c) Klären für eine Dauer, die vom Verlauf der elektrischen Werte, insbesondere des Ohm'sche Widerstands und/oder der Leitfähigkeit, bestimmt wird, bis die Änderungsrate dieser Werte auf ein vorbestimmtes Ausmaß abnimmt,
d) Entwässern, gefolgt von
e) Einbettung in ein Lösungsmittel oder Lösungsmittelgemisch mit einem Brechungsindex, bei dem das Gewebe durchsichtig wird, d.h. einen Brechungsindex > 1.5 hat.

Alternativ zu Schritt d) kann die Probe auch ohne Entwässerung in ein geeignetes, wässriges Medium, z.B. bestehend aus einer konzentrierten Zuckerlösung, aromatische Verbindungen oder Jod-enthaltenden Radiokontrastmittel, eingebettet werden.

Eine Maximaltemperatur der Elektrophoreselösung während des elektrophoretischen Klärens kann in einem Bereich von 20 bis 90 °C gehalten werden. In jedem Fall ist die Temperatur unterhalb eines Siedepunkts der Elektrophoreselösung zu halten. Normalerweise ist es bevorzugt, die Maximaltemperatur der Elektrophoreselösung während des elektrophoretischen Klärens in einem Bereich vom 40 bis 60 °C, das heißt bei ungefähr 50 °C zu halten. Mit einer darüberhinausgehenden Temperatur kann jedoch erreicht werden, dass Anbindungspunkte für Antikörper an Proteine thermisch demaskiert werden, so dass diese Proteine anschließend mit den Antikörpern markiert werden können.

Bei dem erfindungsgemäßen Verfahren resultiert eine Temperaturerhöhung des biologischen Gewebes durch den Eintrag elektrischer Energie, die in Wärme umgewandelt wird. Dieses ist bei der Elektrophorese grundsätzlich unvermeidbar. Bei dem erfindungsgemäßen Verfahren ist jedoch die erreichte Klärung des biologischen Gewebes bezogen auf die eingetragene elektrische Energie besonders groß. Damit fällt es auch besonders leicht, die Temperatur des biologischen Gewebes auf eine Maximaltemperatur in den genannten Bereichen zu beschränken, ohne dass hierfür beispielsweise eine aktive Kühlung der Elektrophoreselösung notwendig wäre.

Während des elektrophoretischen Klärens kann frische Puffersubstanz und/oder frisches Detergens zu der Elektrophoreselösung zugesetzt werden. Alternativ kann die Elektrophoreselösung fortlaufend ausgetauscht werden.

Bei der Durchführung des erfindungsgemäßen Verfahrens kann während des elektrophoretischen Klärens der pH-Wert der Elektrophoreselösung in einem Bereich von 4.5 bis 7.9 gehalten werden, bevorzugt in einem Bereich zwischen 5.0 und 7.9. Der in Folge des angelegten elektrischen Felds fließende elektrische Strom reduziert typischerweise den pH-Wert der Elektrophoreselösung im anodischen Pufferbereich. Im kathodischen Pufferkompartiment steigt dagegen der pH-Wert der Elektrophoreselösung typischerweise an. Um den Wirkungsgrad der Klärung des biologischen Gewebes hochzuhalten, ist es daher sinnvoll, den pH-Wert der Elektrophoreselösung in dem genannten Bereich zu halten. Zu diesem Zweck kann während des elektrophoretischen Klärens frische Puffersubstanz zugesetzt werden. Auch das sich durch das Ausleiten der Mizellen unter Einwirkung des elektrischen Felds verbrauchende Detergens kann während des elektrophoretischen Klärens wieder aufgefüllt werden, indem frisches Detergens zu der Elektrophoreselösung zugesetzt wird. Ein maximaler Wirkungsgrad des erfindungsgemäßen Verfahrens wird erreicht, wenn die Elektrophoreselösung fortlaufend gegen unverbrauchte Elektrophoreselösung ausgetauscht wird.

Bei der Elektrophorese kann eine elektrische Leistung, die an die Elektrophoreselösung und das darin eingetauchte Gewebe abgegeben wird, während des elektrophoretischen Klärens geregelt werden. Die elektrische Leistung kann auch zumindest für einen Teilzeitraum des elektrophoretischen Klärens auf einen festen Wert geregelt werden.

Während des elektrophoretischen Klärens kann eine elektrische Leitfähigkeit der Elektrophoreselösung und des darin eingetauchten biologischen Gewebes erfasst werden. Einzelne Bedingungen der elektrophoretischen Klärung können geändert werden und/oder das elektrophoretische Klären beendet werden, wenn die elektrische Leitfähigkeit oder ihre zeitliche Änderung einen vorgegebenen Grenzwert über- und/oder unterschreitet.

Bei dem erfindungsgemäßen Verfahren kann eine elektrische Leistung, die an die Elektrophoreselösung und das darin eintauchende Gewebe abgegeben wird, während des elektrophoretischen Klärens geregelt werden. Diese Regelung kann abhängig von der Temperatur des biologischen Gewebes bzw. der Elektrophoreselösung erfolgen oder auch auf einen konstanten Wert, der das Einhalten einer bestimmten Maximaltemperatur sicherstellt. Zumindest kann die elektrische Leistung für einen Teilzeitraum des elektrophoretischen Klärens auf einen solchen festen Wert geregelt werden. Wenn die Klärung des biologischen Gewebes weit fortgeschritten und/oder die Elektrophoreselösung bereits zu wesentlichen Teilen verbraucht ist, ist das weitere Einregeln der elektrischen Leistung auf einen festen Wert häufig nicht mehr sinnvoll.

Der Fortschritt des Klärens des biologischen Gewebes kann durch Erfassen einer elektrischen Leitfähigkeit der Elektrophoreselösung und des darin eingetauchten biologischen Gewebes beobachtet werden. Zunächst nimmt der elektrische Widerstand ab, das heißt die elektrische Leitfähigkeit zu, wenn sich die auszuleitenden Mizellen in dem biologischen Gewebe bilden. Mit dem Verbrauch der Elektrophoreselösung und/oder der bereits erfolgten Ableitung der wesentlichen Teile der zu entfernenden Häm-Gruppen und Lipide nimmt die elektrische Leitfähigkeit wieder ab. So können dann bei dem erfindungsgemäßen Verfahren Bedingungen der elektrophoretischen Klärung geändert werden und/oder das elektrophoretische Klären kann beendet werden, wenn der elektrische Widerstand oder seine zeitliche Änderung einen vorgegebenen Grenzwert über- und/oder unterschreitet.

Bevor das biologische Gewebe gemäß dem erfindungsgemäßen Verfahren elektrophoretisch geklärt wird, kann es bereits anderen Behandlungen unterworfen werden. Hierzu zählt, dass das biologische Gewebe fixiert wird, beispielsweise mit Formaldehyd, das die Mobilität der Mizellen anders als ein zur Fixierung verwendetes Hydrogel jedoch nicht einschränkt. Weiterhin zählt das Waschen des biologischen Gewebes, beispielweise mit Wasser oder auch mit der später während des elektrophoretischen Klärens eingesetzten Elektrophoreselösung, zu den möglichen Schritten vor der eigentlichen elektrophoretischen Klärung. Weiterhin wird das biologische Gewebe, bevor es elektrophoretisch geklärt wird, in einer wässrigen alkalischen Lösung inkubiert werden.

Nachdem das biologische Gewebe elektrophoretisch geklärt wurde, kann es für die lichtmikroskopische Untersuchung weiter präpariert werden. Hierzu kann mindestens einer der folgenden Schritte durchgeführt werden:
Das biologische Gewebe wird mit mindestens einem Antikörper inkubiert.

Das biologische Gewebe wird in einer Lösung eines Antikörpers einem elektrischen Feld ausgesetzt.

Das biologische Gewebe wird mit einem organischen Lösungsmittel gewaschen.

Das biologische Gewebe wird mit Xylol oder Dichlormethan gewaschen.

Das biologische Gewebe wird in eine Lösung mit einem Brechungsindex im Bereich von n = 1,4 bis n = 1,6 eingebracht. Der Brechungsindexbereich von n = 1,4 bis n = 1,6, das heißt von etwa n = 1,5 bedeutet, dass die Lösung denselben Brechungsindex wie das geklärte biologische Gewebe aufweist. Damit treten keine Streuungen an den Grenzflächen des biologischen Gewebes bei seiner lichtmikroskopischen Untersuchung auf.

Konkret kann das biologische Gewebe, nachdem es elektrophoretisch geklärt wurde, in eine wässrige Lösung und/oder in eine Zuckerlösung, eine Polyollösung, eine Aromatenlösung oder eine Jod-enthaltenden Radiokontrastmittel-lösung mit einem Brechungsindex in dem genannten Bereich von ungefähr n = 1,5 eingebracht werden. Alternativ kann das biologische Gewebe, nachdem es elektrophoretisch geklärt wurde, in einer Alkoholreihe mit ansteigender Alkoholkonzentration entwässert werden und/oder in ein organisches Lösungsmittel, in Methylsalicylat oder in eine Methylsalicylatlösung mit einem Brechungsindex im Bereich von ungefähr n = 1,5 eingebracht werden.

Mit dem erfindungsgemäßen Verfahren kann eine Klärung biologischer Gewebe zur Herstellung von biologischen Präparaten für eine lichtmikroskopische Untersuchung regelmäßig schon binnen weniger Stunden erreicht werden.

Optional kann die Gewebeprobe vor oder während der Elektrophorese mit Farbstoff oder Antikörper gefärbt werden. Das biologische Gewebe kann in einer Lösung eines Farbstoffs einem elektrischen Feld ausgesetzt werden bevorzugt während der Elektrophorese. Die Färbung erfolgt dann während des elektrophoretischen Klärens, so dass kein separater Schritt für die Färbung notwendig ist.

Die bevorzugte Variante ist die Färbung während der Elektrophorese. Der Einwirkung eines elektrischen Feldes erhöht die Geschwindigkeit des Färbevorgangs, da der Farbstoff oder das gefärbte Biomolekül aufgrund seiner Ladung durch das Gewebe gezogen wird, anstatt es durch Diffusion zu durchdringen.

Bei dem erfindungsgemäßen Verfahren kann der pH bei der Elektrophorese an den pH angepasst werden, bei dem der Farbstoff/Antikörper eine Ladung besitzt, die eine aktive Färbung optimal ermöglicht: Da der elektrophoretische Klärschritt in einem pH-Bereich von basisch (pH > 7.0) über neutral (pH=7.0) bis hinunter zum sauren pH 4.5 durchgeführt werden kann, kann ein pH gewählt werden, bei dem der Farbstoff/Antikörper eine Ladung besitzt, so dass die Einwirkung eines elektrischen Feldes für das gleichzeitige Klären und aktive Färben genutzt werden kann. Der ins saure erweiterte pH-Bereich bietet mehr Möglichkeiten zur Kombination von elektrophoretischem Klären und Färben. Ein weiterer Vorteil einer Färbung unter Einwirkung eines elektrischen Feldes ist, dass das elektrische Feld nicht-gebundene Farbstoffe/Antikörper effizient aus dem Gewebe hinausbefördert. Dieses Vorgehen ersetzt somit langwierige Waschschritte oder die Differenzierung bei einem sogenannten regressiven Färbevorgang. Auf diese Weise wird Zeit und Material gespart. Die Einschränkung auf einen einzigen Elektrophoreseschritt für beide Prozesse minimiert zudem die Joule'sche Erwärmung des Gewebes, die bei jeder Elektrophorese entsteht.

Zur konkreten Durchführung des erfindungsgemäßen Verfahrens kann das Gewebe für das elektrophoretische Klären in einer Reaktionskammer angeordnet werden, die einen um eine Hochachse rotationssymmetrisch ausgebildeten und eine Taillierung aufweisenden, mit der Elektrophoreselösung aufzufüllenden Reaktionsraum, einen nach unten offenen Ringkanal in den Reaktionsraum unterhalb der Taillierung, der an einen nach oben führenden Gasabführkanal angeschlossen ist, eine erste ringförmige Elektrode innerhalb des Ringkanals und/oder in dem Reaktionsraum unterhalb des Ringkanals und eine zweite ringförmige Elektrode in dem Reaktionsraum oberhalb der Taillierung aufweist. Die erste und die zweite Elektrode werden dann an die beiden Ausgänge einer Gleichspannungsquelle angeschlossen, um das biologische Gewebe dem elektrischen Feld auszusetzen. Das Gewebe wird in einem reduzierten freien Querschnitt des Reaktionsraums in der Taillierung angeordnet, wo sich ein annähernd homogenes elektrisches Feld zwischen den Elektroden konzentriert. Über den Gasabführkanal können an der unteren Elektrode gebildete und von dort aufsteigende Gasblasen abgeleitet werden, so dass sich diese nicht im Reaktionsraum sammeln und den elektrischen Stromfluss behindern. Zudem wird die Entstehung von Knallgas verhindert, wenn die Gasblasen durch Hydrolyse gebildeten Wasserstoff enthalten, der sich mit an der oberen Elektrode durch die Hydrolyse gebildetem Sauerstoff vermischen könnte. Die Reaktionskammer kann weitere Merkmale aufweisen, wie sie aus der US 2005/0130317 A1 bekannt sind.

Das erfindungsgemäße Verfahren zur Herstellung transparenter Gewebeproben hat mehrere Vorteile gegenüber den aus dem Stand der Technik bekannten Verfahren und insbesondere gegenüber der basischen Elektrophorese zum Klären einer Gewebeprobe.

Überraschenderweise führt eine Elektrophorese auch bei einem leicht basischen (pH 7.9) über neutralen bis leicht sauren pH (bis hinunter zu pH 4,5), folgend auf eine stark basische Vorbehandlung, ebenfalls zum Klären einer Gewebeprobe. Unterhalb von pH 4,5 werden keine Ladungsträger mehr aus dem Gewebe freigesetzt und das Gewebe wird demnach nicht mehr durchsichtig.

Ebenfalls überraschend ist, dass die Gewebeproben während der Elektrophorese bei leicht basischem über neutralem bis leicht saurem pH nicht aufquellen; einige Gewebeproben zeigen hierbei sogar eine Volumen-Verringerung. Der Verlauf der Gewebe-Größenveränderung während des gesamten Klärverfahrens wird mit einem gleichbleibenden oder abnehmenden Gewebevolumen während der Elektrophorese vorteilhaft unterstützt, da das Gewebe bei der Entwässerung gegenüber der Ausgangsgröße des Gewebes in Formaldehyd schrumpft.

Gegenüber einem Verfahrensverlauf, bei dem einer Gewebevolumen-Zunahme bei einer basischen Elektrophorese eine Abnahme während der Entwässerung folgt, ist dieser neue kontinuierliche Verfahrensverlauf vorzuziehen: die maximale Änderungsrate sowie die gesamte durchgemachte Änderung sind kleiner. Hiermit wird die Problematik der mechanischen Beschädigung von empfindlichen Gewebeproben beseitigt, die durch den Zyklus von Aufquellen und Schrumpfen entsteht.

Da beim erfindungsgemäßen Verfahren die Elektrophorese bei leicht basischem, neutralem oder leicht saurem pH zur Durchsichtigkeit führt, sind neue Anwendungsmöglichkeiten für die gleichzeitige Durchführung von Klären und Färben von Gewebeproben in einem einzigen Elektrophoreseschritt gegeben. Über die Anpassung des pHs der Elektrophoreselösung kann die Richtung und Geschwindigkeit des elektrisch-getriebenen Transports von Farbstoffen und Biomolekülen mit ionisierbaren Gruppen durch die damit hervorgerufenen Ladungsänderungen gesteuert werden. Die gewünschte Kontaktzeit mit dem Gewebe kann so der Klärdauer angepasst werden. Für eine rein passive Färbung vor der Elektrophorese (mit oder ohne anschließender elektrisch getriebener Entfärbung) kann die Zugkraft bei der elektrophoretischen Klärung so begrenzt werden, dass die vorherige Bindung während dieses Schritts nicht verloren geht.

Bei einer Färbung vor der Elektrophorese mit einem geladenen Farbstoff/Antikörper besteht die Gefahr, dass dieser wieder aus dem Gewebe entfernt wird, wenn die elektrische "Zugkraft" höher ist, als die Bindungsstärke für die zu färbenden Gewebekomponenten zulässt. Die wichtigste Anwendung ist daher die Färbung während der Elektrophorese. Der Einwirkung eines elektrischen Feldes erhöht die Geschwindigkeit des Färbevorgangs, da der Farbstoff (oder das gefärbte Biomolekül) aufgrund seiner Ladung durch das Gewebe gezogen wird, anstatt es durch Diffusion zu durchdringen. Allerdings sollte die "Zugkraft" nicht so hoch sein, dass die Interaktionszeit für eine Bindung nicht mehr ausreicht. Die optimale Kondition für das aktive Färben mit einem Farbstoff/Antikörper stimmt meist nicht mit der des elektrophoretischen Klärschritts bei basischem pH überein. Das erfindungsgemäße Verfahren löst somit das Problem des zurzeit fehlenden aber sehr nützlichen pH-Bereichs zwischen 4.5 und 7.9 für die Elektrophorese und ergänzt damit die Möglichkeiten, vor oder während der Elektrophorese, also während des Klärprozesses, effektiv zu färben.

Bei dem erfindungsgemäßen Verfahren können somit beide Elektrophoreseschritte zum Klären und Färben bei leicht saurem, neutralem oder leicht basischem pH durchgeführt werden. Dieses hat die Vorteile, dass
- sich das Gewebevolumen nur in eine Richtung verändert, wobei das Volumen kleiner wird, und die Volumenänderung geringer ausfällt, beide Elektrophoreseschritte zum Klären und Färben auch gleichzeitig in einem einzigen Schritt erfolgen können und
- die elektrophoretisch unterstützte Färbung bei einem für die gefärbten Moleküle und den Färbevorgang optimalen pH durchgeführt werden kann.

Somit verkürzt der Klärvorgang gegenüber der basischen Elektrophorese.

Mit dem erfindungsgemäßen Verfahren kann ein vorteilhaftes Größenprofil während des Klärvorgangs erwirkt werden: das Volumen/die Größe der Gewebeproben nimmt stetig und kontrolliert ab und die Größen-Variationen werden minimiert.

Eine Elektrophorese im leicht sauren (pH>=4,5) bis leicht basischen pH-Bereich führt überraschenderweise und entgegen den Erwartungen zu qualitativ hochwertiger Durchsichtigkeit der Gewebeproben. Für ein besonders schonendes Elektroclearingverfahren ist eine Elektrophorese im leicht sauren (pH>=4,5) bis neutralen pH-Bereich und leicht basischen pH-Bereich vorteilhaft, da sie ein Aufquellen der Probe während der Elektrophorese verhindert und das Schrumpfen hin zur Größe nach der Entwässerung unterstützt bzw. sanft einleitet.

### Beispiele

Der schonende Effekt der sauren/neutralen Elektrophorese wurde anhand der für Größenänderungen besonders anfälligen Gewebe Lunge und Gehirn getestet.

Gewebeproben von menschlichem Gehirn und Schweinelunge wurden bei leicht saurem pH mit der Puffersubstanz 20 mM CAPS (pH 6), und mit der Puffersubstanz 20 mM CHES (pH 5) und bei basischem pH (pH 10) mit der Puffersubstanz 20 mM Tris (Vergleichsbeispiel) elektrophoretisiert. Die Puffersubstanzen wurden als Reinstoff ohne zugemischte andere Puffersubstanzen, Basen oder Säuren zur Einstellung des pHs eingesetzt. Hierdurch wurden die der Flüssigkeit zugeführten Ladungsträger, und damit die Leitfähigkeit der Lösung, minimiert. Alle Lösungen enthielten 1% w/v eines nicht-ionischen Detergens, hier Triton X-100, ein Octylphenolethoxylat. Alle Proben wurden nach dem Klärvorgang durchsichtig.

Die Gewebe wurden in 4% Formaldehyd fixiert und für längere Zeit in der Fixationslösung aufgehoben. Getestet wurden menschliches Gehirn und Schweinelunge, die zwei Jahre gelagert waren. Da Langzeit-fixiertes Gewebe mechanisch empfindlich gegenüber Hantieren und Klären wird, wurde zum Vergleich auch Gehirngewebe, das sieben Jahre lang in Formaldehyd-Lösung aufgehoben wurde, geklärt. Alle Gewebe waren zum Beginn der Behandlung 10 x 10 x 2 mm³ groß.

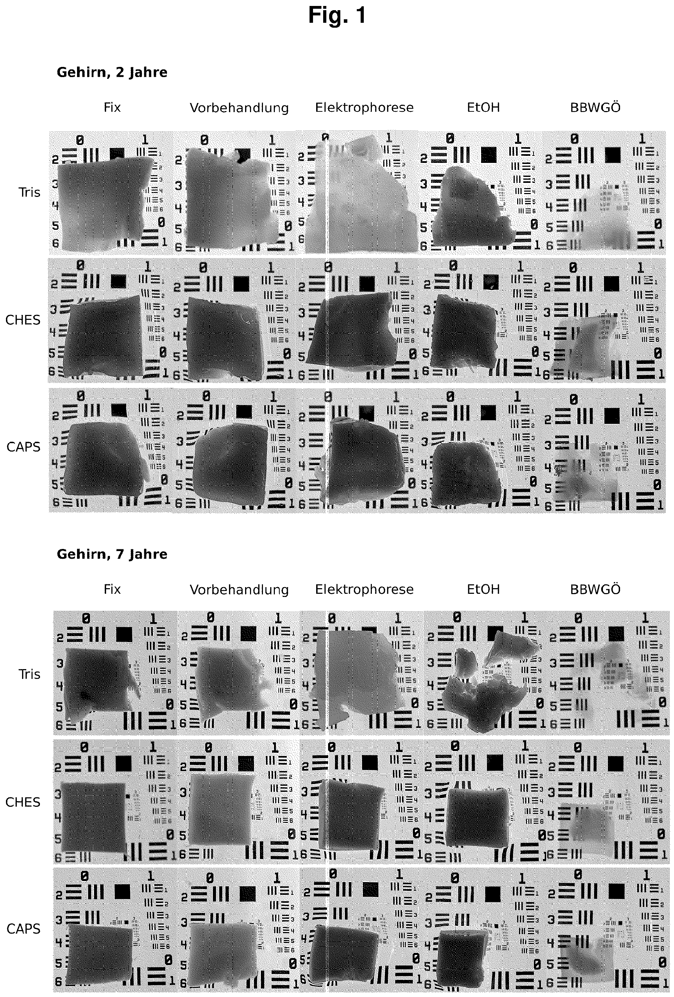

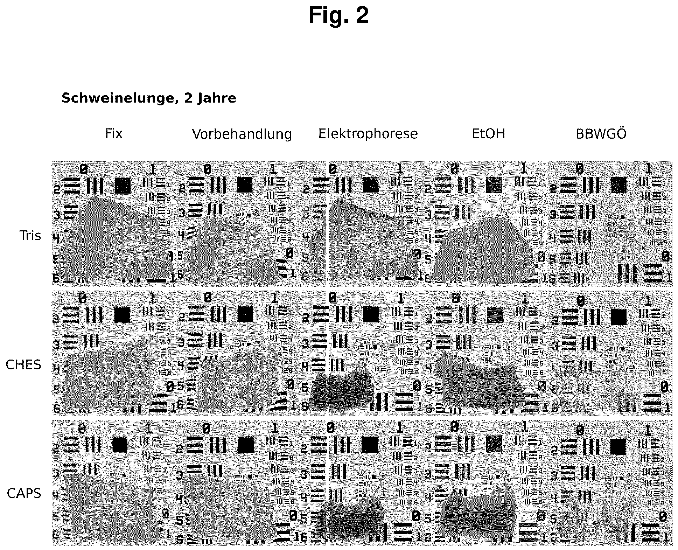

Die Gewebeproben wurden, als Maß für die Volumenänderung, nach jedem Behandlungsschritt gewogen und zur Größenbestimmung fotografiert. Das Gewicht wurde auf das Anfangsgewicht der fixierten Probe normiert. Die wässrigen Lösungen zur Fixierung, zur basischen Vorbehandlung und für die Elektrophorese besitzen eine ähnliche Dichte. Da die Dichte von Ethanol ca. 80% der von Wasser beträgt, ist die Volumenänderung beim Entwässerungsschritt leicht überschätzt, jedoch wegen des unbekannten Trockenanteils der Probe mit diesem Verfahren ohnehin nicht exakt zu bestimmen. Die Fotos der Proben nach den einzelnen Schritten dokumentieren ebenfalls die Größenänderungen (s. Figur 1 und 2).

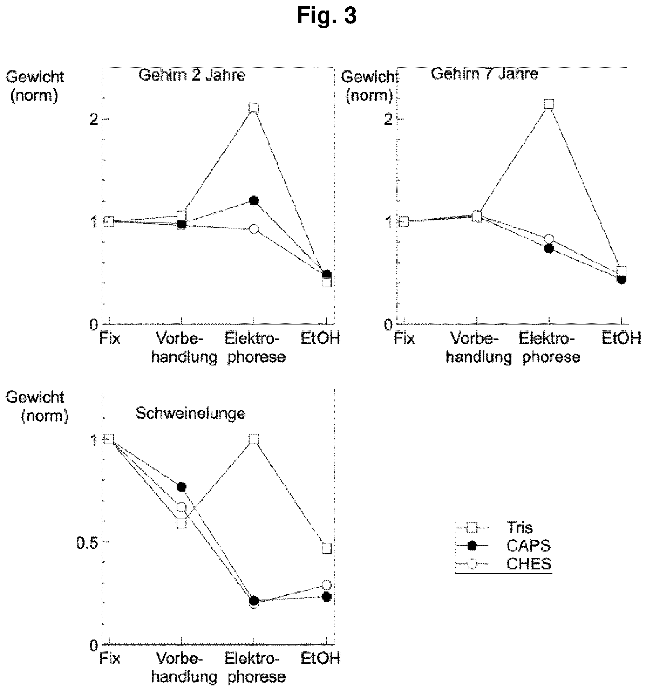

Beide Gewebetypen zeigen eine Volumenverringerung während des Trajektes des fixierten Gewebes ("Fix") zur entwässerten Probe ("EtOH") (s. Fig. 3). Das Gehirngewebe änderte sich während der stark basischen Vorbehandlung kaum. Bei Lungengewebe war dagegen bei diesem Schritt ein Schrumpfen zu beobachten. Beide Gewebe zeigten bei der basischen Elektrophorese in Tris (Vergleichsbeispiel, basische Elektrophorese) eine deutliche und unerwünschte Größenzunahme. Diese Zunahme konnte bei den erfindungsgemäßen Versuchen durch die Nutzung von CAPS und insbesondere CHES vollständig verhindert werden. Bei dem sieben Jahre alten Gehirngewebe war sogar eine Volumenverringerung zu sehen, die sich bei der Entwässerung fortsetzt. Die Nutzung von der erfindungsgemäßen Puffersubstanzen CAPS und CHES bei der Schweinelunge verursachen ebenfalls eine Volumenverringerung. Hier wurde bereits während der Elektrophorese annähernd das Volumen nach der Entwässerung erreicht. Die Nutzung von leicht saurem (CHES) oder (CAPS) Puffer während der Elektrophorese erlaubt daher eine Elektrophorese von Gewebe ohne die Größenzunahme, die bei der Nutzung von basischem Puffer (Tris) auftritt. Die mechanische Belastung durch Aufquellen gefolgt von Schrumpfen konnte eindrucksvoll am Beispiel einer sieben Jahre fixierten Gewebeprobe eines menschlichen Gehirns gezeigt werden.

Dieser vorteilhafte Effekt wurde neben CHES und CAPS bei mehreren unterschiedlichen Puffersubstanzen in den pH-Bereich von 4,5-5.5 (AMPSO, CABS, N,N-Bis-(2-hydroxyethyl)-glycin genannt), pH 5.5-7 (Tricine, TAPS, CAPS Glycyl-Glycine) bis zu neutralem pH (Glycine) getestet und beobachtet. CHES und CAPS zeigen hierbei einen hohen elektrischen Widerstand (geringen Leitfähigkeit), d.h. erlauben einen hohen Spannungsunterschied zwischen den Elektroden, was für den elektrophoretischen Klärvorgang zusätzlich vorteilhaft ist. Im Vergleich ist generell ein Aufquellen von Gewebe bei basischen Puffersubstanzen zu sehen. Bei der Elektrophorese in der Tris-Lösung (Vergleich) zeigt die Probe einen größeren Riss. Die sieben Jahre fixierte Probe des menschlichen Gehirns zerlegte sich danach bei der Entwässerung in mehrere Teile. Bei vergleichbaren Experimenten zeigte sich, dass dieses empfindliche Material oft schon nach der Elektrophorese zerbricht.

Figur 1 zeigt die optischen Größenveränderungen beim Elektroclearing am menschlichen Gehirn. Zwei Gehirnproben von 10x10x2 mm³ wurden, wie vorstehend beschrieben, geklärt. Die Proben wurden zuvor 2 (oberes Panel) oder 7 Jahre (unteres Panel) in Formalin aufgehoben. Die Reihen zeigen den Verlauf der Größen, wenn mit den angegebenen Puffersubstanzen elektrophoretisiert wird. In der angegebenen Reihenfolge der Bilder werden die Proben in Formalin ("Fix"), nach der Vorbehandlung in 1M NaOH, 50% Ethanol und 1% Triton X-100, nach der Elektrophorese in einer Lösung mit 20 mM der angegebenen Puffersubstanz (Tris, CHES und CAPS), nach vollständiger Entwässerung in Ethanol "EtOH"), und nach Einbettung in einer Mischung von Benzylbenzoat und Wintergrünöl ("BBWGÖ") gezeigt.

Figur 2 zeigt die optischen Größenveränderungen beim Elektroclearing an der Schweinelunge. Die Schweinelunge befand sich vor dem Klären 2 Jahre in Formalin. Die Behandlungen und Konditionen entsprechen denen in Figur 1.

Figur 3 zeigt die Größenveränderungen beim Elektroclearen nach Gewicht.

Die Proben aus den Figuren 1 und 2 wurden nach jedem Schritt gewogen. Die Gewichtsänderung dient hierbei als Maß für die vorliegende Größenänderung. Die Gewichte wurden jeweils auf das Anfangsgewicht der Proben im Formalin normiert. Die Ordinaten entsprechen also der relativen Gewichtsänderung. Beide Gewebe zeigen Unterschiede: bei den Gewebeproben aus dem Gehirn beeinflusst die Vorbehandlung das Gewicht kaum, dafür verdoppelt sich bei der basischen Elektrophorese mit Tris (Vergleichsbeispiel) in etwa das Gewicht. Eine Gewichtszunahme wird dagegen mit den beiden anderen erfindungsgemäßen Puffersubstanzen verhindert. Bei der Lunge schrumpft das Gewebe bei der Vorbehandlung, um bei der Elektrophorese mit Tris (Vergleichsbeispiel) wieder etwa zur Ursprungsgröße aufzuquellen. Die Elektrophorese mit den beiden anderen erfindungsgemäßen Puffersubstanzen verhindert auch hier das Aufquellen; sie bewirken dafür ein Schrumpfen etwa bis zum Endgewicht nach der Entwässerung.

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

## Patentansprüche

1. Verfahren zur Herstellung transparenter Gewebeprobe eines biologischen Gewebes zur lichtmikroskopischen Untersuchung, umfassend die Schritte:
a) Inkubation des Gewebes in einer stark basischen Lösung mit einer Säurekonstanten (pKa) ≥ 9,5 und
b) Klären der entwässerten Gewebeprobe durch Elektrophorese, indem das Gewebe in eine wässrige neutrale oder saure Elektrophoreselösung oder leicht basische Elektrophoreselösung eingetaucht und in der Elektrophoreselösung einem elektrischen Feld ausgesetzt wird, wobei die Elektrophoreselösung eine Puffersubstanz in einer Konzentration von 1 bis 100 mol/m³ und mindestens ein nichtionisches Detergens in einer Konzentration von 0,1 bis 10 Gew.-% enthält,
**dadurch gekennzeichnet, dass** die Elektrophoreselösung einen pH-Wert von 4.5-7.9 aufweist und dass die Puffersubstanz eine organische Verbindung mit
mindestens einer Carbonsäuregruppe oder mindestens einer Sulfonsäuregruppe ist und mindestens eine Amingruppe aufweist, und
dass sich der pH-Wert der Elektrophoreselösung durch die Einbringung der Substanz in die wässrige Lösung ergibt.

2. Verfahren gemäß Patentanspruch 1, **dadurch gekennzeichnet, dass** die Elektrophoreselösung einen pH-Wert von pH 5.5 bis 7.9, hat.

3. Verfahren gemäß Patentanspruch 2, **dadurch gekennzeichnet, dass** sich der pH-Wert der Elektrophoreselösung in Wasser spontan einstellt.

4. Verfahren gemäß einem der vorhergehenden Patentansprüche,
**dadurch gekennzeichnet, dass** die Puffersubstanz eine molare elektrische Leitfähigkeit von kleiner als 2 Siemens·cm²/mol bei einer Konzentration von 20mol/m³ aufweist.

5. Verfahren gemäß einem der vorhergehenden Patentansprüche,
**dadurch gekennzeichnet, dass** die Puffersubstanz eine organische Verbindung ist, die genau eine ionisierbare Säure-Gruppe enthält, die pro Molekül ein Proton erzeugen kann, oder die genau eine ionisierbare Base-Gruppe enthält, die pro Molekül ein Hydroxyl-Ion erzeugen kann, und die pro Molekül maximal eine positive und maximal eine negative Ladung und keine weiteren geladenen Gruppen enthält bei dem pH, der durch ihre Lösung in Wasser spontan entsteht, und dass die Elektrophoreselösung mindestens eine Puffersubstanz oder eine Mischung von Puffersubstanzen mit Pufferbasen enthält.

6. Verfahren gemäß Patentanspruch 4 oder 5, **dadurch gekennzeichnet, dass** die Puffersubstanz ausgewählt ist aus der Gruppe bestehend aus Aminocarbonsäuren, Aminosulfonsäuren, Aminocarbonsäuresalzen, Aminosulfonsäuresalzen oder einer Mischung hiervon.

7. Verfahren gemäß Patentanspruch 6, **dadurch gekennzeichnet, dass** die Pfuffersubstanz eine Amino-Alkansulfonsäure, eine Hydroxyalkylaminosulfonsäure, eine Piperidin-Alkansäure, eine Aminosäure, eine N-Hydroxy-Alkyl-Aminocarbonsäure oder ein Aminocarbonsäuresalz oder Amino-Sulfonsäuresalz oder eine Mischung hiervon ist.

8. Verfahren nach einem der vorhergehenden Patentansprüche, **dadurch gekennzeichnet, dass** eine Mischung von Puffersubstanzen mit Pufferbasen enthält, wobei die Puffersubstanz bevorzugt zu mehr als 50 Gew.-%, bevorzugt zu mehr als 75 Gew.-% in der Mischung enthalten ist, bezogen auf das Gesamtgewicht von Puffersubstanz und Pufferbase.

9. Verfahren nach einem der vorhergehenden Patentansprüche, **dadurch gekennzeichnet, dass** das nichtionische Detergens ausgewählt ist aus der Gruppe bestehend aus Polyoxyethylen(20)-sorbitan-monolaurat, Polyoxyethylensorbitan-monooleat, Polyethylenglycol4-tert-octylphenylether, Octylphenoxypolyethoxyethanol, Polyethylenglycol-tert-octylphenylether, n-octyl-beta-D-Glucopyranosid, Octylphenolethoxylat, Decaethylenglykol-mono-dodecylether, Octylphenoxypolyethoxyethanol, sekundäre Alkohol-Ethoxylaten, Betaine, Alkylsulfobetaine oder Mischungen hiervon, bevorzugt das nichtionische Detergens ausgewählt ist aus Octylphenoxypolyethoxyethanol, Decaethylenglykol-monododecylether, sekundäre Alkohol-Ethoxylaten, n-octyl-beta-D-Glucopyranosid, 3-[(3-Cholamidopropyl)dimethylammonio]-1-propanesulfonathydrat und Lauryl Sulfobetain.

10. Verfahren gemäß einem der vorhergehenden Patentansprüche,
**dadurch gekennzeichnet, dass** das Verfahren die Schritte umfasst
0) Fixieren einer Gewebeprobe in Formaldehyd, Glutaraldehyd oder einem anderen vernetzenden Fixativ,
a) Vorbehandeln in einer Lösung mit Alkalimetallhydroxid,
b) Elektrophorese in einer Lösung mit niedrig konzentrierter Puffersubstanz, die ohne Beimischung einer weiteren starken Lauge oder Base mit einer Säurekonstanten (pKa) ≥ 9,5 einen pH-Wert zwischen 4,5 und 7.9 aufweist, in Gegenwart des nicht-ionischen Detergens,
c) Klären für eine Dauer, die vom Verlauf des Ohm'sche Widerstands und/oder der Leitfähigkeit, bestimmt wird, bis die Änderungsrate dieser Werte ein vorbestimmtes Ausmaß abnimmt.

11. Verfahren gemäß Patentanspruch 10, **dadurch gekennzeichnet, dass** das Verfahren um die Schritte der
d) Entwässerung, gefolgt von
e) Einbettung in ein Lösungsmittel oder Lösungsmittelgemisch mit einem Brechungsindex, bei dem das Gewebe durchsichtig wird, erweitert werden kann.

12. Verfahren gemäß einem der vorhergehenden Patentansprüche,
**dadurch gekennzeichnet, dass** das biologische Gewebe mit mindestens einem Farbstoff oder Antikörper gefärbt wird.

13. Verfahren gemäß Patentanspruch 10, **dadurch gekennzeichnet, dass** die Färbung während der Elektrophorese erfolgt.

14. Verfahren gemäß einem der vorhergehenden Patentansprüche,
**dadurch gekennzeichnet, dass** eine Maximaltemperatur der Elektrophoreselösung während des elektrophoretischen Klärens in einem Bereich von 20 bis 90 °C, unterhalb eines Siedepunkts der Elektrophoreselösung, gehalten wird.

## Claims

1. Method for producing a transparent tissue sample of a biological tissue for examination by light microscopy, comprising the steps of:
a) incubation of the tissue in a strongly basic solution with an acid constant (pKa) ≥ 9.5 and
b) clearing of the dehydrated tissue sample by electrophoresis by immersing the tissue in an aqueous neutral or acidic electrophoresis solution or slightly basic electrophoresis solution and exposing it to an electric field in the electrophoresis solution, wherein the electrophoresis solution contains a buffer substance in a concentration of 1 to 100 mol/m³ and at least one nonionic detergent in a concentration of 0.1 to 10 wt.% ( weight percent),
**characterized in that** the electrophoresis solution has a pH of 4.5-7.9 and **in that** the buffer substance is an organic compound with at least one carboxylic acid group or at least one sulfonic acid group and has at least one amine group, and
**in that** the pH of the electrophoresis solution results from the introduction of the substance into the aqueous solution.

2. Method according to patent claim 1, **characterized in that** the electrophoresis solution has a pH of pH 5.5 to 7.9.

3. Method according to patent claim 2, **characterized in that** the pH of the electrophoresis solution in water is adjusted spontaneously.

4. Method according to any of the preceding patent claims,
**characterized in that** the buffer substance has a molar electrical conductivity of less than 2 siemens·cm²/mol at a concentration of 20 mol/m³.

5. Method according to any of the preceding patent claims,
**characterized in that** the buffer substance is an organic compound that contains exactly one ionizable acid group that can produce one proton per molecule, or that contains exactly one ionizable base group that can produce one hydroxyl ion per molecule, and that contains, per molecule, a maximum of one positive and a maximum of one negative charge and no further charged groups at the pH that is spontaneously formed by its dissolution in water, and **in that** the electrophoresis solution contains at least one buffer substance or a mixture of buffer substances with buffer bases.

6. Method according to patent claim 4 or 5, **characterized in that** the buffer substance is selected from the group consisting of aminocarboxylic acids, aminosulfonic acids, aminocarboxylic acid salts, aminosulfonic acid salts or a mixture thereof.

7. Method according to patent claim 6, **characterized in that** the buffer substance is an aminoalkanesulfonic acid, a hydroxyalkylaminosulfonic acid, a piperidine alkanoic acid, an amino acid, an N-hydroxyalkylaminocarboxylic acid or an aminocarboxylic acid salt or aminosulfonic acid salt or a mixture thereof.

8. Method according to any of the preceding patent claims, **characterized in that** a mixture of buffer substances with buffer bases contains, where the buffer substance is present in the mixture preferably to an extent of more than 50 wt.%, preferably to an extent of more than 75 wt.%, based on the total weight of buffer substance and buffer base.

9. Method according to any of the preceding patent claims, **characterized in that** the nonionic detergent is selected from the group consisting of polyoxyethylene (20) sorbitan monolaurate, polyoxyethylene sorbitan monooleate, polyethylene glycol 4-tert-octylphenyl ether, octylphenoxypolyethoxyethanol, polyethylene glycol tert-octylphenyl ether, n-octyl-β-D-glucopyranoside, octylphenol ethoxylate, decaethylene glycol monododecyl ether, octylphenoxypolyethoxyethanol, secondary alcohol ethoxylates, betaines, alkyl sulfobetaines or mixtures thereof; preferably, the nonionic detergent is selected from octylphenoxypolyethoxyethanol, decaethylene glycol monododecyl ether, secondary alcohol ethoxylates, n-octyl-β-D-glucopyranoside, 3-[(3-cholamidopropyl)dimethylammonio]-1-propanesulfonate hydrate and lauryl sulfobetaine.

10. Method according to any of the preceding patent claims,
**characterized in that** the method comprises the steps of
0) fixation of a tissue sample in formaldehyde, glutaraldehyde or another crosslinking fixative,
a) pretreatment in a solution with alkali metal hydroxide,
b) electrophoresis in a solution with a low concentration of buffer substance that, without addition of a further strong alkali or base with an acid constant (pKa) ≥ 9.5, has a pH between 4.5 and 7.9, in the presence of the nonionic detergent,
c) clearing for a period determined by the course of the ohmic resistance and/or the conductivity until the rate of change of these values decreases to a predetermined extent.

11. Method according to patent claim 10, **characterized in that** the method can be expanded by the steps of
d) dehydration, followed by
e) embedding in a solvent or solvent mixture with a refractive index at which the tissue becomes transparent.

12. Method according to any of the preceding patent claims,
**characterized in that** the biological tissue is stained with at least one dye or antibody.

13. Method according to patent claim 10, **characterized in that** the staining is performed during the electrophoresis.

14. Method according to any of the preceding patent claims,
**characterized in that** a maximum temperature of the electrophoresis solution during the electrophoretic clearing is kept in a range of 20 to 90°C, below a boiling point of the electrophoresis solution.

## Revendications

1. Procédé de fabrication d'un échantillon de tissu transparent d'un tissu biologique destiné à un examen par microscopie optique, comprenant les étapes suivantes :
a) l'incubation du tissu dans une solution fortement basique ayant une constante d'acidité (pKa) ≥ 9,5, et
b) la clarification de l'échantillon de tissu déshydraté par électrophorèse, le tissu étant immergé dans une solution d'électrophorèse aqueuse neutre ou acide ou dans une solution d'électrophorèse légèrement basique et étant exposé à un champ électrique dans la solution d'électrophorèse, la solution d'électrophorèse contenant une substance tampon à une concentration de 1 à 100 mol/m³ et au moins un détergent non ionique à une concentration de 0,1 à 10 % en poids,
**caractérisé en ce que** la solution d'électrophorèse présente une valeur de pH de 4,5 à 7,9 et **en ce que** la substance tampon est un composé organique comprenant au moins un groupe acide carboxylique ou au moins un groupe acide sulfonique et comprenant au moins un groupe amine, et
**en ce que** la valeur du pH de la solution d'électrophorèse résulte de l'introduction de la substance dans la solution aqueuse.

2. Procédé selon la revendication 1, **caractérisé en ce que** la solution d'électrophorèse présente une valeur de pH de 5,5 à 7,9.

3. Procédé selon la revendication 2, **caractérisé en ce que** la valeur du pH de la solution d'électrophorèse s'établit spontanément dans l'eau.

4. Procédé selon l'une des revendications précédentes, **caractérisé en ce que** la substance tampon présente une conductivité électrique molaire inférieure à 2 Siemens·cm²/mol à une concentration de 20 mol/m³.

5. Procédé selon l'une des revendications précédentes, **caractérisé en ce que** la substance tampon est un composé organique contenant exactement un groupe acide ionisable pouvant générer un proton par molécule ou contenant exactement un groupe basique ionisable pouvant générer un ion hydroxyde par molécule, et qui comporte par molécule au maximum une charge positive et au maximum une charge négative et ne comporte aucun autre groupe chargé au pH qui s'établit spontanément par sa dissolution dans l'eau, et **en ce que** la solution d'électrophorèse contient au moins une substance tampon ou un mélange de substances tampons avec des bases tampons.

6. Procédé selon la revendication 4 ou 5, **caractérisé en ce que** la substance tampon est choisie dans le groupe constitué par des acides aminocarboxyliques, des acides aminosulfoniques, des sels d'acides aminocarboxyliques, des sels d'acides aminosulfoniques ou un mélange de ceux-ci.

7. Procédé selon la revendication 6, **caractérisé en ce que** la substance tampon est un acide amino-alcanesulfonique, un acide hydroxyalkylaminosulfonique, un acide pipéridine-alcanoïque, un acide aminé, un acide N-hydroxyalkyl-amino-carboxylique ou un sel d'acide aminocarboxylique ou un sel d'acide aminosulfonique, ou un mélange de ceux-ci.

8. Procédé selon l'une des revendications précédentes, **caractérisé en ce qu'**il contient un mélange de substances tampons avec des bases tampons, la substance tampon étant présente de préférence à plus de 50 % en poids, de préférence à plus de 75 % en poids dans le mélange, par rapport au poids total de la substance tampon et de la base tampon.

9. Procédé selon l'une des revendications précédentes, **caractérisé en ce que** le détergent non ionique est choisi dans le groupe constitué par le monolaurate de polyoxyéthylène(20)-sorbitane, le mono-oléate de polyoxyéthylène sorbitane, l'éther 4-tert-octylphénylique de polyéthylène glycol, l'octylphénoxypolyéthoxyéthanol, l'éther tert-octylphénylique de polyéthylène glycol, le n-octyl-β-D-glucopyranoside, l'éthoxylate d'octylphénol, l'éther monododécylique de décaéthylène glycol, l'octylphénoxypolyéthoxyéthanol, des éthoxylates d'alcools secondaires, des bétaïnes, des alkylsulfobétaïnes ou des mélanges de ceux-ci, de préférence le détergent non ionique étant choisi parmi l'octylphénoxypolyéthoxyéthanol, l'éther monododécylique de décaéthylène glycol, des éthoxylates d'alcools secondaires, le n-octyl-β-D-glucopyranoside, l'hydrate de 3-[(3-cholamidopropyl)diméthylammonio]-1-propanesulfonate et la laurylsulfobétaïne.

10. Procédé selon l'une des revendications précédentes, **caractérisé en ce que** le procédé comprend les étapes suivantes :
0) la fixation d'un échantillon de tissu dans du formaldéhyde, du glutaraldéhyde ou un autre agent fixateur réticulant,
a) le prétraitement dans une solution contenant un hydroxyde de métal alcalin,
b) l'électrophorèse dans une solution contenant une substance tampon faiblement concentrée qui, sans ajout d'une autre lessive forte ou base présentant une constante d'acidité (pKa) ≥ 9,5, présente une valeur de pH comprise entre 4,5 et 7,9, en présence du détergent non ionique,
c) la clarification pendant une durée déterminée à partir de l'évolution de la résistance ohmique et/ou de la conductivité, jusqu'à ce que le taux de variation de ces valeurs diminue jusqu'à une ampleur prédéterminée.

11. Procédé selon la revendication 10, **caractérisé en ce que** le procédé peut être complété par les étapes suivantes :
d) la déshydratation, suivie de
e) l'inclusion dans un solvant ou un mélange de solvants ayant un indice de réfraction pour lequel le tissu devient transparent.

12. Procédé selon l'une des revendications précédentes, **caractérisé en ce que** le tissu biologique est coloré à l'aide d'au moins un colorant ou anticorps.

13. Procédé selon la revendication 10, **caractérisé en ce que** la coloration est effectuée pendant l'électrophorèse.

14. Procédé selon l'une des revendications précédentes, **caractérisé en ce qu'**une température maximale de la solution d'électrophorèse pendant la clarification électrophorétique est maintenue dans une plage de 20 à 90 °C, en dessous du point d'ébullition de la solution d'électrophorèse.
